Europäisches Patentamt

**European Patent Office**

**Office européen des brevets**

(11) Publication number: **0 189 864**
**B1**

(12) **EUROPEAN PATENT SPECIFICATION**

(45) Date of publication of patent specification: **29.11.89**

(21) Application number: **86100948.8**

(22) Date of filing: **24.01.86**

(51) Int. Cl.⁴: **B 01 J 47/00,** B 01 J 20/24,
C 12 N 11/12

(54) **Process for increasing enzyme adsorption.**

(30) Priority: **25.01.85 US 694867**

(43) Date of publication of application:
**06.08.86 Bulletin 86/32**

(45) Publication of the grant of the patent:
**29.11.89 Bulletin 89/48**

(84) Designated Contracting States:
**AT BE CH DE FR GB IT LI LU NL SE**

(56) References cited:
**DE-A-2 356 532**
**GB-A- 896 439**
**US-A-3 634 394**
**US-A-4 110 164**
**US-A-4 355 117**

(73) Proprietor: **NABISCO BRANDS, Inc.**
**Nabisco Brands Plaza**
**Parsippany New Jersey 07054 (US)**

(72) Inventor: **Antrim, Richard L.**
**610 13th Avenue North**
**Clinton Iowa (US)**
Inventor: **Harris, Donald W.**
**3333 Lost Bridge Road**
**Decatur Illinois (US)**

(74) Representative: **Hoffmann, Klaus, Dr. rer. nat.
et al**
**Hoffmann . Eitle & Partner Patentanwälte**
**Postfach 81 04 20 Arabellastrasse 4**
**D-8000 München 81 (DE)**

## Description

This invention relates to the field of enzyme immobilization. More specifically, the invention provides a process for increasing the adsorption capacity of granular derivatized cellulosic ion exchange composites.

In food processing and other commercial applications, the use of microbial or fungal enzymes adsorbed onto or bonded to inert carriers to provide immobilized biological catalysts has largely superseded older methods wherein soluble enzymes or whole cells of microorganisms were utilized. In general, the use of immobilized enzymes provides a number of significant advantages over the older methods. The major advantage is that the immobilized enzymes are adaptable for use in continuous conversion processes. Thus, a more efficient use of the enzyme is attained and the contact time between the enzyme and the substrate is reduced, thereby resulting in an improved product quality and a reduction in enzyme and production costs.

Cellulose occurs in nature as a linear polymer comprised of anhydroglucose units joined together by β-1,4 glucosidic bonds. Each anhydroglucose unit contains three free hydroxyl groups capable of reacting with appropriate reagents to form insoluble cellulose derivatives which, due to their relative inertness, large surface area, and open, porous structure, have a high adsorptive or ion-exchange capacity for protein molecules.

The preparation and utilization of ion exchange enzyme adsorbents derived from cellulose are known in the art. Peterson and Sober, J.A.C.S. 78, 751 (1956) and Guthrie and Bullock, I/EC, 52, 935 (1960) described methods for preparing adsorptive cellulose products which could be utilized to separate or purify enzymes and other proteins. Tsumura et al., Nippon Shakuhin Kogyo Gakkaishi. 14, (12), (1967) disclosed binding glucose isomerase to DEAE-Sephadex.

U.S. Patent No. 3,708,397 to Sipos relates to a process for immobilizing glucose isomerase on basic anion exchange celluloses. U.S. Patent No. 3,823,133 to Hurst et al. is directed to a method for preparing cationic cellulose ethers having a high adsorptive capacity for enzymes and other proteinaceous materials. U.S. Patent No. 3,838,007 to van Velzen sets forth a process in which an enzyme preparation is obtained in particulate form. U.S. Patent Nos. 3,788,945 and 3,909,354, both to Thompson et al., disclose continuous processes for converting glucose to fructose by passing a glucose-containing solution through fixed or fluidized beds containing glucose isomerase bound to various cellulose products. U.S. Patent No. 3,947,325 to Dinelli et al. is directed to the preparation of cellulose-containing englobed enzymatic material. The cellulose is formed from an emulsion comprising an aqueous enzyme solution and nitrocellulose. U.S. Patent No. 3,956,065 to Idaszak et al. is concerned with a continuous process for converting glucose to fructose whereby a glucose-containing solution is passed through a bed comprising a cellulose derivative having glucose isomerase immobilized thereon and non-porous or granular polystyrene beads. The beads inhibit packing and channeling of the bed when such is used in flow reactors. Peska et al. in an article entitled "Ion Exchange Derivatives of Bead Cellulose," Die Angewandte Makromolekulare Chemie, 53, pp. 73—80 (1976), describes several derivatized celluloses prepared in bead form.

U.S. Patent Nos. 4,110,164 and 4,168,250 both to Sutthoff et al. relate to agglomerated fibrous ion exchange cellulose composites and processes for preparing the same. In these processes a hydrophobic polymer is combined with fibrous cellulose which has previously been derivatized to impart ion exchange properties thereto. Although these composites perform satisfactorily in a number of applications, their ion exchange capability and capacity for adsorbing or binding glucose isomerase are not as great as desired. Moreover, the economics of these processes are such as to make the production of the composites more costly than is preferred.

U.S. Patent No. 4,355,117 to Antrim et al. provides a process for preparing an agglomerated DEAE-cellulose composite which overcomes many of shortcomings discussed above and is the preferred process for preparing the composite useful in practicing this invention.

This invention provides a process for preparing an agglomerated DEAE cellulose composite capable of adsorbing or binding charged macromolecules wherein a DEAE-cellulose is agglomerated with a hydrophobic polymer to form a granular composite, the improvement comprising treating the composite with an aqueous medium at elevated temperature and for sufficient time to increase the adsorption or binding capacity of the composite.

This invention further provides a process for increasing the adsorption or binding capacity of a granular DEAE-cellulose composite for charged macromolecules comprising:

a) agglomerating a cellulose with a hydrophobic polymer to form a granular composite;

b) derivatizing the agglomerated cellulose in the composite with a derivatizing agent to impart ion exchange properties thereto, at least a portion of the derivatized cellulose being free to adsorb charged macromolecules;

c) maintaining the derivatized composite in granular forms; and

d) treating the granular derivatized composite with an aqueous medium at an elevated temperature for a sufficient period of time to increase the macromolecular adsorption capacity of said derivatized composite.

Cellulose can be derivatized to provide ion exchange materials having high loading capacities in regard to adsorbing or immobilizing macromolecules. For this purpose, the cellulose may be derivatized to provide ion exchange materials having either anion or cation exchange capabilities, depending upon the charge present on the material to be adsorbed. When the material to be adsorbed is glucose isomerase, the cellulose will advantageously be derivatized to the anion exchange form since in this form its loading capacity for this enzyme is extremely high. Typically, to produce the anion exchange form the agglomerated cellulose will be treated with appropriate reagents to form, among others, the di- and tri-ethylaminoethyl celluloses, such as DEAE-cellulose and TEAE-cellulose, and the cellulose derivatives of epichlorohydrin and triethanolamine, such as ECTEOLA-cellulose. Background information and methods for derivatizing cellulose are disclosed in U.S. Patent No. 3,823,133 to Hurst *et al.* The present invention is limited to the treatment of granular DEAE-cellulose.

Although the following description and Examples are primarily directed to the utilization of agglomerated ion exchange cellulose to adsorb and immobilize glucose isomerase, it is also demonstrated that the agglomerated material has the ability to adsorb other proteinaceous material and it is contemplated that the agglomerated material will have the capability of adsorbing other charged macromolecules such as nucleic acids and the like, and further, would be capable of recovery of said molecules from a variety of substances such as food waste streams, e.g. recovery of protein from milk whey, meat processing streams and vegetable processing streams, reduction of BOD from waste streams, etc.

Due to the high loading capacity of ion exchange cellulose preparations containing glucose isomerase, when such are utilized in industrial applications, relatively small reactors may be employed to convert large quantities of glucose to fructose.

Additionally, because of this high loading capacity, the substrate and the resulting product are maintained under isomerization conditions for only a short period. These isomerization conditions, generally, are conducive to production of small amounts of unwanted by-products due to the reactive nature of the fructose, and thus, the longer the period the fructose is maintained under such conditions, the greater the amounts of unwanted by-products produced. Thus, the high loading capacity of ion exchange cellulose results in the substrate being isomerized to the desired degree in a short time, thereby decreasing the period during which the fructose component is maintained under isomerization conditions. However, such preparations containing non-granular ion exchange cellulose suffer from the disadvantage of "packing" and, therefore, such are usually utilized in shallow beds to avoid the development of problems due to excessive backpressure. Even when shallow beds are utilized, there is the possibility of channeling occurring whereby the substrate is not contacted to the desired degree with the bound or immobilized glucose isomerase. Although certain immobilized glucose isomerase preparations have been developed to minimize these problems, they generally suffer other disadvantages, e.g., their enzyme capacity or activity per unit volume is not as high as is desired, and/or they are not as economical as ion exchange cellulose.

In practicing the present invention a number of polymers may be utilized to agglomerate the cellulose. Exemplary of these are polymers such as melamine formaldehyde resins, epoxy resins, polystyrene and the like. The preferred polymer is polystyrene.

In U.S. Patent Nos. 4,110,164 and 4,168,250 it is disclosed that when cellulose which has been derivatized to provide an ion exchange material is agglomerated with a hydrophobic polymer under suitable conditions, such cellulose retains its capacity to immobilize or bind glucose isomerase. The preferred process taught for preparing the composites comprises treating alkali-cellulose with a solution of diethylaminoethyl chloride hydrochloride (DEC) and then agglomerating the derivatized ion exchange cellulose formed thereby with polystyrene. Due to the solubility of polystyrene in the DEC reaction mixture, however, it would be anticipated that the cellulose could not be efficiently derivatized if the agglomerates were formed prior to derivatization of the cellulose.

As mentioned previously, it was discovered by *Antrim et al.* and disclosed in U.S. Patent No. 4,355,117 that cellulose can be efficiently derivatized in the presence of the hydrophobic polymer by controlling process conditions during derivatization so as to prevent the polymer from becoming solubilized in the derivatizing solution. Thus, it was found that by adding the derivatizing material at a controlled rate to a water suspension of the agglomerate under alkaline conditions, the hydrophobic polymer component of the granular composite does not become solubilized to a significant degree.

A further unexpected finding was that when the cellulose was derivatized following agglomeration thereof, the cellulose composite may be derivatized to a higher degree and thus have a greater ion exchange capacity than the agglomerated cellulose composite produced by the process of the prior art wherein the cellulose is derivatized before agglomerization. While the ion exchange capacity of the agglomerated fibrous cellulose composite of that invention may vary widely, typically the ion exchange capacity should be at least about 0.1 meq g$^{-1}$ and preferably at least about 0.2 meq g$^{-1}$.

It has now been surprisingly discovered that following the preparation of derivatized composite, a treatment with a hot aqueous medium can dramatically increase the adsorption capacity of the composite. By treatment with a hot aqueous medium is meant a treatment at elevated temperatures in a medium such as tap water, deionized water or dilute salt solutions of up to about 7000 umhos of conductivity or more, preferably about 1000 umhos of conductivity.

In preparing the composite it is preferred to employ the method of Antrim *et al.* as described in U.S. Patent No. 4,355,117 the contents of which are incorporated herein by reference.

Specifically, two types of derivatized granular cellulose were prepared, Type I—GDC contained alumina as a densification agent while Type II—GDC contained titanium dioxide as the densification agent.

In preparing each composite the following formulation procedure was used. An agglomerate was prepared by mixing 30 parts CEPO S—100 cellulose (Swedish Cellulose Powder and Woodflower Milk Ltd, Gothenburg, Sweden) and 20 parts of weighting agent (either alumina in the case of Type I or titanium dioxide in the case of Type II) and compounding the mixture with 50 parts polystyrene on a heated (180°—200°C) twin roll compounder for a period of 10 minutes. After cooling, the compounded composite was ground and sized to 40—80 mesh.

Into one of two 2-liter, four necked resin pots equipped with stirrer, thermometer with a Therm-O-Watch Regulator, condenser, and heating mantle was placed 300 grams of either Type I or Type II granular cellulose ($GC_I$ or $GC_{II}$) and 942 ml of deionized water. With stirring was added 300 grams of anhydrous sodium sulfate, then 82 gms of 50% sodium hydroxide. To each reaction mixture heated to 40°C was added using a Technicon Pump 117 grams of 50% diethylaminoethyl chloride hydrochloride (DEC) reagent. The reagent was added over a 2 hour period. The mixture was stirred at 40°C for 30 minutes, then 62 grams of NaOH was added to each resin and additional 117 grams of DEC reagent was added over a two hour period. The reaction mixtures were then heated to 60°C for approximately 30 minutes, then cooled and the pH adjusted to 6.5 using 30% $H_2SO_4$. The products are designated as granular derivatized cellulose Type I ($GDC_I$) and granular derivatized cellulose Type II ($GDC_{II}$).

The following examples illustrate the subject invention but should not be construed to limit same.

Example 1

This example illustrates the increase in adsorption capacity for the enzyme glucose isomerase realized when granular derivatized cellulose ($GDC_I$) prepared with alumina weighting agent was treated with water at about 90°C.

Using a U.S. No. 60 mesh stainless steel sieve, 50 g f.b. of dried $GDC_I$ was screened using about 24 liters of water from a shower head. The screened $GDC_I$ was dewatered with a sintered glass funnel and vacuum flask, and then bottled and labeled Sample A.

To 600 ml of deionized water contained in a 1000 ml stainless steel beaker was placed 21.7 g d.b. of the above screened $GDC_I$. The beaker was placed in a water bath heated to about 90°C and the mixture was stirred in the 90°C water bath for thirty minutes.

The hot mixture was filtered using a coarse sintered glass funnel and suction flask, and then the GDC solids were placed back into the 1000 ml stainless beaker. To the beaker was added a second 600 ml of deionized water and the mixture was treated with stirring a second time for thirty minutes in the 90°C water bath.

The hot mixture was filtered as before, and then the GDC solids were treated a third time with 600 ml of deionized water for thirty minutes in the 90°C water bath. After a final filtration the dewatered, thrice treated $GDC_I$ was bottled and labeled Sample B.

The adsorption capacity of the treated GDC for glucose isomerase was determined using a single point procedure. Thus, into a 140 ml plastic flask with lid was placed 4.8358 g f.b. (2.0262 g d.b.) of the treated $GDC_I$ (Sample B) and 50.0 ml of deionized water. To this mixture was added 0.5 ml of 1 M tris buffer, pH 7.0 and 1.25 ml of glucose isomerase (5412 (IGIU/ml) containing 6765 IGIU of activity. The glucose isomerase may be purified from extracts containing the enzyme by procedures well-known in the art, such purification procedures include but are not limited to centrifugation, salt (e.g. ammonium sulfate) precipitation, and column (e.g. ion exchange or affinity) chromatography. An International Glucose Isomerase Unit (IGIU) is that amount of enzyme which will convert 1 micromole of glucose to fructose per minute in a solution containing 2 moles glucose, 0.02 moles $MgSO_4$, 0.001 moles $CoCl_2$ per liter at a pH of 6.84 to 6.85 (0.2 M sodium maleate) at a temperature of 60°C. Sodium chloride was added to give a conductivity of about 9.5 × $10^5$ umhos and the mixture was agitated 1 hr. The pH was adjusted to 7.1 using dilute HCl and the sample was agitated 4 hrs. After settling, a sample of the clear liquid was diluted 1:10 and analyzed via the Technicon Assay as described by Lloyd, *et al. In: Cereal Chemistry* 49(5): 544—553 (1972). It was found that the clear liquid contained a total of 803 IGIU of glucose isomerase activity. Thus the 2.0262 g d.b. of GDC adsorbed a total of 5960 IGIU of glucose isomerase or 2940 IGIU/g d.b. of $GDC_I$.

Using the same procedure for the starting GDC (Sample A) and the same enzyme, an adsorption capacity of 1955 IGIU/g $GDC_I$ d.b was obtained. The high temperature treatment thus resulted in a 50% increase in the enzyme adsorption capacity of the $GDC_I$.

Example 2

This example illustrates the increase in enzyme adsorption capacity realized when granular derivatized cellulose ($GDC_{II}$) prepared with titanium dioxide weighting agent was treated with water at about 90°C.

A 65.5 g f.b. sample (34.5 g d.b.) of $GDC_{II}$ was stirred in a beaker containing about 600 ml of deionized water (room temperature) and then screened using a shower of water, on a U.S. No. 60 mesh stainless steel sieve using about 24 liters of water. The washed GDC was dewatered on a sintered glass funnel, bottled and labeled Sample A. This sample served as a control; there was no 90°C treatment step.

Another 34.5 g d.b. sample of $GDC_{II}$ was placed, along with 600 ml of deionized water, into a stainless steel beaker. The beaker was placed in a hot water bath at approximately 90°C and the slurry was stirred at this bath temperature for 1/2 hour. The resulting product was washed on a U.S. No. 60 mesh stainless steel sieve to remove fines, using about 24 liters of water. The sample was dewatered on a sintered glass funnel, bottled and labeled Sample B.

Another 34.5 g d.b. sample of $GDC_{II}$ was placed, along with 600 ml of deionized water, into a stainless steel beaker and treated as above in the 90°C water bath for 1/2 hour. The solids were filtered, resuspended in another 600 ml of deionized water and treated in the 90°C water bath for 1/2 hour a second time. The product was washed on a U.S. No. 60 mesh sieve to remove fines then dewatered using a sintered glass funnel, bottled and labeled Sample C.

Another 34.5 g d.b. sample of $GDC_{II}$ was treated three times with 600 ml portions of deionized water for 1/2 hour each in the 90°C water bath. The resulting product was then washed on a U.S. No. 60 mesh sieve to remove fines, using about 24 liters of water. The $GDC_{II}$ product was dewatered, bottled and labeled Sample D.

The four GDC products were weighed and analyzed for adsorption capacity of glucose isomerase enzyme. Results are presented below.

| Sample | A | B | C | D |
|---|---|---|---|---|
| GI Adsorption Capacity (IGIU/g $GDC_{II}$, d.b.) | 2492 | 3455 | 3456 | 3660 |

The 90°C treatment resulted in an average increase in glucose isomerase enzyme adsorption capacity of 45%.

## Example 3

This example illustrates the adsorption capacity from samples of granular cellulose (GC) first derivatized with varying levels of DEC reagent then treated with water at about 90°C.

### Derivatization

Into six 2-liter, four necked resin pots equipped with stirrer, thermometer with Therm-O-Watch Regulator, condensor and heating mantle was placed 300 g of standard GC (compound from polystyrene, CEPO S—100 cellulose and titanium dioxide weighting agent; 50%, 30%, and 20%, respectively prepared as described above) and 942 ml of deionized water. With stirring was added 300 g of anhydrous sodium sulfate then 82 g of 50% sodium hydroxide. To each reaction mixture heated to 40°C was added using a Technicon Pump, the quantity of 50% diethylaminoethyl chloride hydrochloride (DEC) reagent equivalent to either 50%, 60%, 70%, 80%, 90% or 100% the standard level of DEC reagent. The 50% DEC reagent was added over a 2 hour period.

The reaction mixtures were stirred at 40°C for 30 minutes then 62 g of 50% NaOH was added to each resin pot and the second half of 50% DEC reagent was added over a two hour period.

The reaction mixtures were heated to 60°C for approximately 30 minutes then cooled and the pH was adjusted to 5 using 30% $H_2SO_4$. Presented below are the reaction conditions used for each product.

| Exp. No. | Initial GC Water (g) | (g) | $Na_2SO_4$ | 50% NaOH (g) 1st Add'n. | 2nd Add'n. | % of Std. | 50% DEC Reagent % 1st Add'n. (g) | % 2nd Add'n. (g) |
|---|---|---|---|---|---|---|---|---|
| 1 | 942 | 300 | 300 | 82 | 62 | 100 | 117 | 117 |
| 2 | 942 | 300 | 300 | 82 | 62 | 90 | 105 | 105 |
| 3 | 942 | 300 | 300 | 82 | 62 | 80 | 93.6 | 93.6 |
| 4 | 942 | 300 | 300 | 82 | 62 | 70 | 81.9 | 81.9 |
| 5 | 942 | 300 | 300 | 82 | 62 | 60 | 70.2 | 70.2 |
| 6 | 942 | 300 | 300 | 82 | 62 | 50 | 58.5 | 58.5 |

### Screening & Washing

The reaction products were screened through a U.S. No. 30 mesh sieve and collected on a U.S. No. 60 mesh sieve using about 24 liters of water each from a shower head. The product from each reaction (i.e., U.S. #30 to #60 mesh size) was stirred in 2000 ml each of water and washed on a U.S. No. 60 mesh sieve using the shower head and 24 liters of water. The solids were again dispersed in 2000 ml of water and again washed on the U.S. No. 60 mesh sieve using the shower head and 24 liters of water each. The six reaction products were dewatered on a sintered glass funnel.

High Temperature Treatment

Into 1,000 ml stainless steel beakers were placed 50.0 grams d.b. of each product and 600 ml of deionized water. The beakers were placed in a 90°C water bath and the GDC slurries were stirred for 2 hours at the 90°C bath temperature. The solids were recovered by hot filtration using a coarse sintered glass funnel then suspended in 600 ml of deionized water and again stirred for 2 hours in the 90°C water bath. The resulting hot GDC slurries were filtered using a coarse sintered glass funnel then the solids were resuspended in 600 ml of deionized water and again stirred for 2 hours in the 90°C bath. After filtration the thrice treated GDC products were screened on a U.S. No. 60 mesh sieve using about 24 liters of water and a shower head. The products were dewatered, and capacity for gluocose isomerase measured. Results are presented below:

| | Level of 50% DEC Reagent (%) | | | | | |
|---|---|---|---|---|---|---|
| Sample No. | 100 | 90 | 80 | 70 | 60 | 50 |
| Adsorption Capacity after 90°C treatment (IGIU/g GDC, d.b.) | 3130 | 2714 | 2538 | 2538 | 2468 | 1953 |

As may be expected the adsorption capacity for glucose isomerase decreased as a function of the DEC reagent level. However, the GDC product prepared using only 60% of the standard level of DEC reagent had an adsorption capacity equivalent to GDC derivatized with 100% of the standard DEC level but not treated at 90°C. See Example 2.

Example 4

This example illustrates the effect of treatment temperature on adsorption capacity of GDC.

A 300 g d.b. sample of GDC$_{II}$ was screened and washed on a 60 mesh screen essentially as described in Example 2. The screened GDC was dewatered on a sintered glass funnel and blended thoroughly. Samples of the blended GDC$_{II}$ were taken and suspended in deionized water (10 ml/g d.b.). The samples were stirred for two hours at various temperatures ranging from 50 to 100°C. The GDC$_{II}$s were then collected by filtration on sintered glass funnels. A sample of each GDC$_{II}$ was removed for analysis and the remaining GDC$_{II}$ were resuspended in fresh water for a second two-hour treatment. After the second treatment the GDC$_{II}$s were collected by filtration, sampled for analysis and resuspended in water for a third two-hour treatment. After the third treatment the GDC$_{II}$s were washed with cold water on a 60 mesh screen, dewatered by filtration, and thoroughly blended. The retained samples from the single and double treatments were also screened, dewatered and blended.

The adsorption capacity of each of the above GDC$_{II}$s was determined with glucose isomerase essentially as described in Example 1 except that multiple point adsorptions using varying amounts of GDC$_{II}$ with a constant amount of enzyme were used. Adsorption capacity was calculated by plotting isomerase activity adsorbed vs. weight of GDC$_{II}$ added. The results are summarized in the following table.

Adsorption Capacities of Treated GDC$_{II}$s

| Treatment Temperature °C | Number of Treatments | Adsorption Capacity IGIU/g d.b. |
|---|---|---|
| Control (untreated) | 0 | 2550 |
| 50 | 1 | 2465 |
| | 2 | 2762 |
| | 3 | 2830 |
| 60 | 1 | 2489 |
| | 2 | 2841 |
| | 3 | 3303 |
| 70 | 1 | 2592 |
| | 2 | 2756 |
| | 3 | 3147 |
| 80 | 1 | 3058 |
| | 2 | 2913 |
| | 3 | 3460 |
| 90 | 1 | 2858 |
| | 2 | 3491 |
| | 3 | 3438 |
| 100 | 1 | 4876 |
| | 2 | 5216 |
| | 3 | 5158 |

At all temperatures tested adsorption capacity was improved by multiple treatments. The adsorption capacity increased with increasing treatment temperature, such that the adsorption capacity was almost doubled by two or three treatments at 100°C.

Example 5

This example illustrates the effect of high temperature treatment on improving the adsorption capacity of GDC$_{II}$ for a protein other than glucose isomerase.

To determine if the hot water treatment would improve the adsorption capacity for other proteins as well as for glucose isomerase, a 0.2% solution of bovine serum albumin (BSA) (Sigma, Fraction V) was prepared by dissolving the protein in 10 mM Tris buffer, pH 7.0. The conductivity of this solution was adjusted to 9000 umhos by addition of sodium chloride.

Samples of washed and screened GDC$_{II}$ and of GDC$_{II}$ which had been treated three times at 100°C as described in Example 4 were used to determine adsorption capacity for BSA. Weighted portions of each GDC$_{II}$ were suspended in 50 ml aliquots of BSA solution and agitated for 60 minutes. The GDC$_{II}$ was allowed to settle by gravity and samples of the clear supernates were taken to determine soluble BSA concentration by ultraviolet absorbance at 280 nm. The adsorption capacity was calculated by difference in soluble BSA concentration before and after GDC$_{II}$ addition.

The 100° treated GDC$_{II}$ adsorbed 19.7 mg BSA/g d.b. compared to an adsorption of only 11.6 mg BSA/g d.b. for the untreated GDC$_{II}$. Thus, the 100° treatment increased the adsorption capacity for bovine serum albumin by about 70%.

Example 6

This example demonstrates the effect of high temperature treatment on other carriers known to adsorb glucose isomerase.

To determine if the improvement in adsorption capacity after hot water treatment of GDC is a general property of isomerase adsorbants, several carriers known to adsorb glucose isomerase were treated for two hours with water at 90°C. Isomerase adsorption capacity was then determined with the untreated and treated carriers. Fibrous DEAE-cellulose (Whatman DE—23), a macroporous polystyrene quaternary amine resin (Amberlite IRA—900, Rohm & Haas), and a macroporous polyphenolic tertiary amine resin (Duolite A—568, Diamond Shamrock) were tested. Ten gram portions of each resin were suspended in 100 ml of deionized water and stirred for two hours at 90°C. The resins were collected and dewatered on sintered glass funnels and portions were used to determine isomerase adsorption capacity essentially as described in Example 4. No difference in isomerase adsorption capacity of the treated vs. untreated carriers was found for IRA—900, IRA—932, or Duolite A—568. The adsorption capacity for Whatman DE—23 was reduced from 3407 IGIU/g for the untreated carrier to 2429 IGIU/g for the treated carrier. Thus, hot water treatment did not improve the adsorption capacity of any of the other non-GDC resins cited.

7

EP 0 189 864 B1

### Example 7

This example demonstrates that high temperature treatments with either tap water or dilute salt solution are also effective in increasing adsorption capacity of GDC.

To compare the effect of deionized water, tap water (620 umhos conductivity), and dilute sodium chloride solution (5000 umhos conductivity), samples of GDC II were treated three times at 90°C with each solution essentially as described in Example 4. The adsorption capacity of the treated GDC's was determined with glucose isomerase, as described in Example 1. The following results were obtained.

| Medium | Adsorption Capacity (IGIU/g d.b.) |
|---|---|
| None | 2550 |
| Deionized Water | 3438 |
| Tap Water | 3170 |
| Salt Solution | 4070 |

Treatment with tap water was almost as effective as treatment with deionized water in increasing GDC adsorption capacity. The dilute salt solution (0.05 NaCl) was more effective than either deionized or tap water.

### Example 8

This example demonstrates the effect of conductivity (salt concentration) on treatment of GDC.

To determine the effect of conductivity on the efficiency of treatment, samples of GDC—412 were treated three times at 100°C with sodium chloride solutions of various conductivity essentially as described in Example 4. The adsorption capacity of each of the treated GDC's was determined with glucose isomerase, as described in Example 1. The following results were obtained.

| Conductivity (umhos) | Adsorption Capacity (IGIU/g d.b.) |
|---|---|
| 52 | 4259 |
| 1000 | 4642 |
| 3000 | 4260 |
| 7000 | 3980 |
| Control (untreated) | 2750 |

In all cases the treatment improved the adsorption capacity of the GDC over that of the untreated control. Optimum improvement was seen after treatment with 1000 umhos salt solution (~0.01N NaCl).

### Claims

1. A process for producing an agglomerated DEAE cellulose composite capable of adsorbing or binding charged macromolecules wherein a DEAE-cellulose is agglomerated with a hydrophobic polymer to form a granular composite, characterized in that the granular DEAE-cellulose is treated with an aqueous medium at elevated temperature for sufficient time to increase the adsorption or binding capacity of the composite.

2. A process for increasing the adsorption or binding capacity of a granular DEAE-cellulose composite for charged macromolecules comprising:

a) agglomerating a cellulose with a hydrophobic polymer to form a granular composite;

b) derivatizing the agglomerated cellulose in the composite with a derivatizing agent to impart ion exchange properties thereto, at least a portion of the derivatized cellulose being free to adsorb charged macromolecules;

c) maintaining the derivatized composite in granular form; and

d) treating the granular derivatized composite with an aqueous medium at an elevated temperature for a sufficient period of time to increase the macromolecular adsorption capacity of said derivatized composite.

3. The process according to Claim 2 wherein said derivatizing agent is diethylaminoethyl chloride.

4. The process according to Claim 2 wherein said hydrophobic polymer is polystyrene.

8

5. The process according to Claim 2 wherein said composite has present a densification agent.

6. The process according to Claim 5 wherein said densification agent is selected from the group consisting of powdered metal oxides, silicates and mixtures thereof.

7. The process according to Claim 6 wherein said densification agent is alumina or titanium oxide.

8. The process according to Claim 1 or 2 wherein the treatment temperature is from 50°C to 100°C.

9. The process according to Claim 1 or 2 wherein the treatment time is from one-half to 3 hours.

10. The process according to Claim 1 or 2 wherein said aqueous medium is selected from the group consisting of tap water, deionized water and a dilute salt solution.

11. The process according to Claim 1 or 2 wherein said treatment is repeated up to three times or more.

12. The process according to Claim 1 or 2 wherein said macromolecule is a protein.

13. The process according to Claim 1 or 2 wherein said protein is an enzyme.

14. The process according to Claim 13 wherein said enzyme is glucose isomerase.

**Patentansprüche**

1. Verfahren zur Herstellung eines agglomerierten DEAE-Cellulose-Verbundkörpers, der in der Lage ist, zugeführte Makromoleküle zu adorbieren oder zu binden, bei dem DEAE-Cellulose mit einem hydrophoben Polymer unter Ausbildung eines körnigen Verbundkörpers agglomeriert wird, dadurch gekennzeichnet, daß die körnige DEAE-Cellulose mit einem wäßrigen Medium bei erhöhter Temperatur während einer ausreichend Zeit unter Erhöhung der Adsorptions- oder Bindungskapazität des Verbundkörpers behandelt wird.

2. Verfahren zum Erhöhen der Adsorptions- oder Bindungskapazität von körnigen DEAE-Cellulose-Verbundkörpern für zugeführte Makromoleküle, umfassend:

a) Agglomerieren einer Cellulose mit einem hydrophoben Polymer unter Ausbildung eines körnigen Verbundkörpers,

b) Derivatisieren der agglomerierten Cellulose in dem Verbundkörper mit einem Derivatisierungsmittel, wodurch diesem Ionenaustauscheigenschaften verliehen werden, wobei wenigstens ein Teil der derivatisierten Cellulose frei ist, um die zugegebenen Makromoleküle zu adsorbieren,

c) Beibehalten des derivatisierten Verbundkörpers in körniger Form und

d) Behandeln des körnigen derivatisierten Verbundstoffes mit einem wäßrigen Medium bei erhöhter Temperatur während einer ausreichenden Zeit unter Erhöhung der makromolekularen Adsorptionsfähigkeit des deriviatisierten Verbundstoffes.

3. Verfahren nach Anspruch 2, bei dem das Derivatisierungsmittel Diethylaminoethylchlorid ist.

4. Verfahren gemäß Anspruch 2, bei dem das hydrophobe Polymer Polystyrol ist.

5. Verfahren gemäß Anspruch 2, bei dem in dem Verbundstoff ein Verdichtungsmittel vorliegt.

6. Verfahren gemäß Anspruch 5, bei dem das Verdichtungsmittel ausgewählt ist aus der Gruppe bestehend aus pulverisierten Metalloxiden, Silikaten und Mischungen daraus.

7. Verfahren gemäß Anspruch 6, bei dem das Verdichtungsmittel Aluminiumoxid oder Titanoxid ist.

8. Verfahren gemäß Anspruch 1 oder 2, bei dem die Behandlungstemperatur 50°C bis 100°C beträgt.

9. Verfahren gemäß Anspruch 1 oder 2, bei dem die Behandlungszeit eine halbe bis drei Stunden beträgt.

10. Verfahren gemäß Anspruch 1 oder 2, bei dem das wäßrige Medium ausgewählt ist aus der Gruppe bestehend aus Leitungswasser, entionisiertem Wasser und einer verdünnten Salzlösung.

11. Verfahren gemäß Anspruch 1 oder 2, bei dem die Behandlung bis zu dreimal oder mehr wiederholt wird.

12. Verfahren gemäß Anspruch 1 oder 2, bei dem das Makromolekül ein Protein ist.

13. Verfahren gemäß Anspruch 1 oder 2, bei dem das Protein ein Enzym ist.

14. Verfahren gemäß Anspruch 13, bei dem das Enzym Glucoseisomerase ist.

**Revendications**

1. Procédé de fabrication d'un composite de DEAE-cellulose agglomérée, capable d'adsorber ou de lier des macromolécules chargées, suivant lequel une DEAE-cellulose est agglomérée avec un polymère hydrophobe pour former un composite granulaire, caractérisé par le fait que la DEAE-cellulose granulaire est traitée par un milieu aqueux, à température élevée, pendant une période de temps suffisante pour augmenter la capacité d'adsorption ou de liaison du composite.

2. Procédé pour augmenter la capacité d'adsorption ou de liaison d'un composite de DEAE-cellulose granulaire pour des molécules chargées, consistant à:

a) agglomérer une cellulose avec un polymère hydrophobe pour former un composite granulaire;

b) transformer la cellulose agglomérée en un dérivé, dans le composite, par un agent de transformation en dérivé, pour lui conférer des propriétés d'échange d'ions, au moins une partie de la cellulose transformée en dérivé étant libre d'adsorber des macromolécules chargées;

c) maintenir le composite transformé en dérivé sous une forme granulaire; et

d) traiter le composite transformé en dérivé, granulaire, par un milieu aqueux, à une température élevée, pendant une période de temps suffisante pour augmenter la capacité d'adsorption macromoléculaire dudit composite transformé en dérivé.

3. Procédé selon la revendication 2, dans lequel ledit agent de transformation en dérivé est le chlorure de diéthylaminoéthyle.

4. Procédé selon la revendication 2, dans lequel ledit polymère hydrophobe est le polystyrène.

5. Procédé selon la revendication 2, dans lequel ledit composite renferme un agent de densification.

6. Procédé selon la revendication 5, dans lequel ledit agent de densification est choisi dans le groupe constitué par les oxydes et les silicates métalliques pulvérulents et leurs mélanges.

7. Procédé selon la revendication 6, dans lequel ledit agent de densification est l'alumine ou l'oxyde de titane.

8. Procédé selon l'une des revendications 1 et 2, dans lequel la température de traitement va de 50°C à 100°C.

9. Procédé selon l'une des revendications 1 et 2, dans lequel le temps de traitement va d'une demi-heure à 3 heures.

10. Procédé selon l'une des revendications 1 et 2, dans lequel ledit milieu aqueux est choisi dans le groupe constitué par l'eau de distribution, l'eau désionisée et une solution saline diluée.

11. Procédé selon l'une des revendications 1 et 2, dans lequel ledit traitement est répété jusqu'à trois fois ou davantage.

12. Procédé selon l'une des revendications 1 et 2, dans lequel ladite macromolécule est une protéine.

13. Procédé selon l'une des revendications 1 et 2, dans lequel ladite protéine est une enzyme.

14. Procédé selon la revendication 13, dans lequel ladite enzyme est la glucose isomérase.